# EUROPÄISCHE PATENTSCHRIFT

(11) **EP 4 363 035 B1**
(45) Veröffentlichungstag und Bekanntmachung des Hinweises auf die Patenterteilung: **15.04.2026**
(21) Anmeldenummer: 22741736.7
(22) Anmeldetag: 30.06.2022
(51) Int. Cl.: A61M 60/109, A61M 60/279, A61M 60/37, A61M 60/441, A61M 60/835, A61M 60/849, A61M 1/16, A61M 1/36, F04B 53/16, F04B 43/12

(54) **KLEMMELEMENT FÜR EINE PUMPVORRICHTUNG**
CLAMPING ELEMENT FOR A PUMP DEVICE
ÉLÉMENT DE SERRAGE POUR UN DISPOSITIF DE POMPE

(30) Priorität: 30.06.2021 DE 102021116833
(43) Veröffentlichungstag der Anmeldung: 08.05.2024
(73) Patentinhaber: Fresenius Medical Care Deutschland GmbH, 61352 Bad Homburg (DE)
(72) Erfinder: KIRSTGEN, Udo, 61352 Bad Homburg (DE)
(74) Vertreter: Breuninger, Marcus
(86) Internationale Anmeldenummer: PCT/EP2022/068169
(87) Internationale Veröffentlichungsnummer: WO 2023/275303

(56) Entgegenhaltungen:
- GB-A- 2 290 582
- US-A- 5 533 877

## Beschreibung

### Technisches Gebiet

Die Erfindung betrifft ein Klemmelement zum Fixieren eines Schlauches gemäß dem Oberbegriff von Anspruch 1, eine Pumpvorrichtung für eine medizinische Vorrichtung gemäß dem Oberbegriff von Anspruch 8, eine Blutbehandlungsvorrichtung gemäß dem Oberbegriff von Anspruch 13 sowie einen Schlauchsatz gemäß dem Oberbegriff von Anspruch 14.

### Hintergrund

Bekannt aus dem Stand der Technik sind Schlauchpumpen, auch Peristaltikpumpen genannt. Diese Art von Pumpen findet häufig im medizinischen Bereich zur Förderung von Fluiden Anwendung. Insbesondere werden Schlauchpumpen bei Dialysemaschinen verwendet, so dass das geförderte Fluid beispielsweise Blut, Dialysierflüssigkeit, eine Medikamentenlösung zur Einbringung von Medikamenten in das Blut des Patienten, beispielsweise Heparin, Citratlösung, Eisenkomplexlösungen oder andere Medikamente, oder auch Substituat ist. Dabei wird beispielsweise durch an einem Rotor angebrachte Okklusionsrollen ein in einem Schlauch geführtes Fluid gefördert. Der Schlauch wird dabei zwischen dem Rotor und einem Stator eingelegt. Durch die Okklusionsrollen wird das Fluid in Umfangsrichtung gefördert, wobei in Förderrichtung stromab einer Okklusionsrolle Überdruck und stromauf einer Okklusionsrolle Unterdruck im geförderten Fluid herrscht. Eine Schlauchpumpe weist typischerweise 2 oder 4 dieser radial am Rotor angeordneten Okklusionsrollen auf. Durch diese in einer Schlauchpumpe herrschenden Drücke ergibt sich sowohl eine Belastung auf das zu fördernde Fluid aber auch auf den verwendeten Schlauch. Insbesondere kann es im Eingangs- oder Ausgangsbereich der Pumpe zu hohen Belastungen auf den Schlauch kommen.

Da eine Veränderung eines Rotors oder Stators einer Schlauchpumpe einen hohen konstruktiven Aufwand mit sich bringt und die Lebensdauer von medizinischen Vorrichtungen aufgrund der Zulassungsvoraussetzungen lange ist, ist es wünschenswert Veränderungen an Rotor oder Stator zu vermeiden.

Ebenso können Verstärkungselemente am Schlauch selbst nur sehr bedingt vorgenommen werden. Medizinische Schläuche zur Verwendung bei Dialysemaschinen müssen besonders hohen hygienischen Standards sowie Sicherheitsstandards genügen. Zudem unterliegen Schläuche als Einmalartikel einem hohen Kostendruck.

Die EP1763636B1 beschreibt eine Rollenpumpe mit einem Rotor, welcher einen bogenförmig in das Pumpenbett eingelegten Schlauch beaufschlagt oder durch ein Anschlussstück in der Rollenpumpe fixiert wird. Das Gehäuse der Rollenpumpe weist seitlich zwei Clipsaufnahmen unterschiedlicher Form für das in seiner Form an diese angepasste Anschlussstück auf, wobei eine Clipsaufnahme in Form eines Rundbogens und die andere Clipsaufnahme eckig ausgebildet ist.

Weiterer relevanter Stand der Technik ist beispielsweise in den Dokumenten GB2290582 A und US5533877 A offenbart.

Eine Aufgabe der vorliegenden Erfindung ist es die Belastung auf das geförderte Fluid sowie den verwendeten Schlauch zu verringern.

### Zusammenfassung der Erfindung

Die erfindungsgemäße Aufgabe wird durch ein Klemmelement mit den Merkmalen des Anspruchs 1, eine Pumpvorrichtung mit den Merkmalen des Anspruchs 8 sowie eine Blutbehandlungsvorrichtung mit den Merkmalen des Anspruch 13 gelöst.

Das Klemmelement zum Fixieren eines Schlauches weist eine erste Führung und eine zweite Führung zum Aufnehmen eines Schlauches auf. Dabei laufen die zwei Führungen aufeinander zu, und das Klemmelement ist derart ausgebildet, dass es mit einer Pumpvorrichtung verbindbar ist, so dass eine Schlauchführung durch die zwei Führungen am Pumpeneingang und Pumpenausgang ausbildbar ist.

Durch die Anordnung der Führungen, welche vorzugsweise in einer Richtung zu dem Rotor weiter voneinander beabstandet sind, als in einer Richtung, weiter entfernt des Rotors, wird vorzugsweise eine Schlauchführung erreicht, bei welcher der Schlauch in einem Winkel kleiner 120°, vorzugsweise kleiner 90°, vorzugsweise kleiner 45° zu einer Tangente am Rotor eingeführt wird. Die Tangente kann dabei beispielsweise am Einführungspunkt des Schlauches auf dem Rotor gebildet werden. Beispielsweise kann die Tangente an dem Punkt gebildet werden, an welchem der Schlauch am Umfang des Rotors erstmals geführt wird.

Dabei kann das Klemmelement bei einer bevorzugten Ausgestaltung derart mit einer Pumpvorrichtung verbindbar ausgebildet sein, dass eine Schlauchführung zumindest am Pumpeneingang und/oder Pumpenausgang im Wesentlichen tangential zu einem Rotor der Pumpvorrichtung verläuft. Hierdurch wird der Schlauch tangential zu dem Rotor der Pumpvorrichtung zugeführt. Die Krafteinwirkung des Rotors auf den Schlauch ist somit nicht sprunghaft, sondern kontinuierlich ansteigend. Zudem lassen sich durch die tangentiale Zuführung starke Scherkräfte im Schlauch selbst unterbinden.

Der zu verwendende Schlauch ist insbesondere ein Schlauch für medizinische Anwendungen, insbesondere für eine Dialysebehandlung, beispielsweise ein Schlauch für den extrakorporalen Kreislauf zur Führung von Blut. Für medizinische Anwendungen vorgesehene Schläuche müssen dabei besonders hohen hygienischen Standards genügen. Dabei sind diese Schläuche häufig aus einem PVC-Material und weisen vorzugsweise keine Beschichtung auf. Beschichtungen könnten sich während einer Behandlung lösen und die Gefahr bestehen, dass Partikel in den Kreislauf gelangen. Dadurch sind die am Schlauch aufbringbaren Verstärkungselemente beschränkt. Durch die tangentiale Zuführung des Schlauches können durch konstruktive Mittel hohe Belastungen auf den Schlauch sowie das geführte Fluid, etwa Blut, verhindert werden.

Die zwei Führungen können dabei als Vertiefungen, etwa rillenförmig mit einem gleichbleibenden Querschnitt oder sich veränderndem Querschnitt, als Vorsprünge, etwa mit seitlichen Beschränkungen zur Schlauchpositionierung oder als geometrische Aufnahmen oder Beschränkungen zur Schlauchpositionierung auf dem Klemmelement ausgebildet sein. Dabei können die Führungen über die gesamte Oberfläche des Klemmelements oder nur über einen Teilbereich der Oberfläche verlaufen. Ebenso können die Führungen einen Radius aufweisen, welcher dem Außenradius des einlegbaren Schlauches entspricht. Ebenso kann der Radius aber auch größer oder kleiner oder sich über den Verlauf der Führungen verändernd ausgebildet sein. Die Führungen können ebenso derart ausgebildet sein, dass ein einsetzbarer Schlauch nur an zumindest zwei Stellen, das heißt punktförmig aufgenommen wird, ebenso können die Führungen derart ausgebildet sein, dass die Aufnahme des Schlauches linienförmig oder flächig erfolgt.

Ebenso kann die Form der Führungen in einer Draufsicht auf das Klemmelement nahezu dreieckförmig ausgebildet sein. Durch die Führungen wird die Position eines einlegbaren Schlauches in Bezug zu dem Klemmelement bestimmt. Die Form der Führungen kann dabei identisch oder verschieden ausgebildet sein. Die Führungen auf dem Klemmelement sind dabei derart ausgebildet, dass bei einem Einsetzen des Klemmelements in eine Pumpvorrichtung zumindest am Pumpeneingang oder am Pumpenausgang eine Schlauchführung entsteht, mittels welcher der Schlauch in einer definierten Lage der Pumpvorrichtung zugeführt oder von dieser weggeführt wird. Die vollständige Fixierung des Schlauches kann dabei vollständig durch das Klemmelement oder in einem Zusammenspiel aus Klemmelement und Pumpvorrichtung bewirkt werden. Insbesondere trägt das Klemmelement mit Hilfe der Führungen zu einer Fixierung des Schlauchs durch eine Klemmfunktion bei. Ein Teil einer Fixierung des Schlauchs wird herkömmlich bereits von der Pumpvorrichtung selbst übernommen.

Das Klemmelement gemäß der Offenbarung ist bevorzugt derart ausgebildet, dass die zwei Führungen einen zumindest im Bereich einer Pumpvorrichtung einschließlich Pumpeneingang und Pumpenausgang einstückigen Schlauch zu einer Schlaufe formen. Somit ist es unnötig, wie bei der eingangs zitierten EP1763636B1 einen bogenförmigen bzw. schlaufenförmigen Schlauch in das Pumpenbett einzulegen, der als vom restlichen Schlauch getrennter Wegwerfartikel ausgebildet ist. Darüber hinaus ist gemäß dieser bevorzugten Ausführungsform der Offenbarung eine Klebe- und/oder Steckverbindung in ein Anschlussstück bzw. das Klemmeelement unnötig. Vielmehr ermöglicht das Klemmelement ein klebe- und steckfreies Fixieren eines einstückigen bzw. durchgehenden Schlauches. Bei der EP1763636B1 wird das Anschlussstück eingeklemmt, jedoch nicht der Schlauch. Der Schlauch selbst bzw. Schlauchstücke jedoch wird bei der EP1763636B1 in das Anschlussstück gesteckt und/oder geklebt.

Die zwei Führungen können weiter in Relation zueinander derart ausgebildet sein, dass bei einer gedachten Verlängerung der Führungen ein Winkel zwischen diesen von 5° bis 90°, bevorzugt von 10° bis 60°, noch bevorzugter von 25° bis 50°, ausgebildet wird.

Nach einer Weiterbildung kann das Klemmelement zumindest teilweise elastisch ausgebildet sein, insbesondere kann ein erster Seitenabschnitt des Klemmelements elastisch ausgebildet sein.

Durch eine zumindest teilweise elastische Ausbildung des Klemmelements kann ein Verbinden des Klemmelements mit der Pumpvorrichtung erleichtert werden. Dabei kann das Klemmelement an zumindest einer Seite derart elastisch ausgebildet sein, so dass ein leichtes Einsetzen und Herausnehmen des Klemmelement aus der Pumpvorrichtung gegeben ist. Das Klemmelement kann damit aus einem elastischen Material, beispielsweise einem elastischen Kunststoff ausgebildet sein. Mit anderen Worten kann ein thermoplastisches Elastomer sowie Silikon oder Gummi eingesetzt werden.

Nach einer Weiterbildung kann das Klemmelement zumindest ein Verbindungselement zum Verbinden mit einer Pumpvorrichtung, insbesondere einen Vorsprung, bevorzugt einen stiftförmigen Vorsprung, aufweisen. Dabei kann das Verbindungselement an einem zweiten Seitenabschnitt, welcher dem ersten Seitenabschnitt gegenüberliegt, ausgebildet sein.

Das Verbindungselement kann derart ausgebildet sein, dass es ein lösbares Verbinden des Klemmelements mit der Pumpvorrichtung ermöglicht. Mit anderen Worten ist das Verbindungselement eine derart geometrisch an dem Klemmelement ausgebildetes Element, so dass bei einem Verbinden des Klemmelements mit der Pumpvorrichtung zumindest die Lage in einer Dimension zwischen Klemmelement und Pumpvorrichtung eindeutig definiert ist. Mit anderen Worten kann das Verbindungselement eine nutförmige Aussparung am Klemmelement oder ein federförmiger Vorsprung sein.

Ebenso kann das Verbindungselement als ein stiftförmiger Vorsprung oder eine stiftförmige Aussparung am Klemmelement ausgebildet sein. Bevorzugt ist das Verbindungselement an einem zweiten Seitenabschnitt ausgebildet, welcher dem ersten Seitenabschnitt gegenüberliegt. Hierdurch wird die Montage des Klemmelements weiter vereinfacht. Insbesondere kann hierdurch zuerst das Klemmelement mit dem zweiten Seitenabschnitt zuerst eingesetzt werden und eine Verbindung mittels des Verbindungselements erreicht werden, so dass durch den elastischen ersten Seitenabschnitt ein vollständiges Einsetzen des Klemmelements leicht möglich ist.

Erfindungsgemäß sind die zwei Führungen auf einem ersten Abschnitt ausgebildet, wobei der erste Abschnitt vorzugsweise um eine Drehachse eines zweiten Abschnitts rotierbar sein kann.

Mit anderen Worten weist das Klemmelement zumindest zwei Abschnitte auf, welche zueinander beweglich ausgebildet sind. Dabei kann an dem zweiten Abschnitt eine Drehachse ausgebildet oder gelagert sein. Der erste Abschnitt, auf welchem die Führungen ausgebildet sind, kann dabei um diese Drehachse rotierbar mit dem zweiten Abschnitt verbunden sein. Dabei kann die Drehachse integral mit dem zweiten Abschnitt ausgebildet sein. Beispielsweise kann die Drehachse zwei stiftförmige Vorsprünge aufweisen, mit welchen der erste Abschnitt verbindbar, beispielweise aufsteckbar ist.

Alternativ kann in dem zweiten Abschnitt eine Drehachse gelagert sein, um welcher der erste Abschnitt rotierbar ist. Mittels einer derartigen Verbindung kann die Lage zwischen dem ersten Abschnitt und dem zweiten Abschnitt veränderbar ausgebildet sein. Insbesondere kann der erste Abschnitt derart kippbar auf dem zweiten Abschnitt angeordnet sein, so dass der erste Abschnitt die Lage der Führungen relativ zu dem zweiten Abschnitt und damit relativ zu der Pumpvorrichtung verändert.

Nach einer Weiterbildung kann der erste Abschnitt vorzugsweise mittels einer Feder, insbesondere einer Spiralfeder, in einer ersten Position vorgespannt sein, wobei in der ersten Position der erste Abschnitt von dem zweiten Abschnitt zumindest teilweise beabstandet ist.

Nach einer Weiterbildung kann die Rotationsachse des ersten Abschnitts an dem zweiten Abschnitt angeordnet sein.

Nach einer Weiterbildung kann die Rotationsachse an dem zweiten Abschnitt und die Spiralfeder auf der Rotationsachse gelagert sein.

Mit anderen Worten weist das Klemmelement zwei Abschnitte auf, welche zueinander beweglich angeordnet sind. Dabei kann an dem ersten und/oder zweiten Abschnitt ein Federelement ausgebildet sein, welches den ersten Abschnitt gegen den zweiten Abschnitt vorspannt. Dabei kann das Federelement aber auch mit der am zweiten Abschnitt angeordneten Drehachse verbunden angeordnet sein. Insbesondere kann eine Spiralfeder auf der Drehachse positioniert sein, so dass der erste Abschnitt gegen den zweiten Abschnitt verkippt angeordnet ist. Damit kann beispielsweise eine auf einen eingesetzten Schlauch wirkende Kraft veränderbar ausgebildet sein. So kann der erste Abschnitt während des Einsetzens des Schlauches in Richtung des zweiten Abschnitts gedrückt werden. Ebenso können hierdurch Schläuche verschiedener Durchmesser sicher gehalten werden, indem der Abstand zwischen dem ersten und zweiten Abschnitt veränderbar ausgebildet ist.

Nach einer Weiterbildung können die erste und/oder zweite Führung rillenförmig ausgebildet sein. Ebenso oder alternativ können die erste Führung und/oder die zweite Führung den Schlauch an zwei gegenüberliegenden Seiten des Schlauchs aufnehmen.

Mit anderen Worten kann zumindest eine Führung rillenförmig ausgebildet sein, wobei diese im Querschnitt einen Kreissektor von kleiner 180° aufspannt, insbesondere einen Kreissektor von kleiner 90°, insbesondere einen Kreissektor von kleiner 45°, insbesondere einen Kreissektor von kleiner 30°, aufspannt. Ebenso kann zumindest eine Führung rillenförmig ausgebildet sein, wobei diese im Querschnitt einen Kreissektor von 180° oder größer aufspannt. Alternativ kann zumindest eine Führung zwei sich gegenüberliegende Seitenwände aufweisen und nahezu U-förmig ausgebildet sein. Alternativ kann zumindest eine der Führungen eine Omega-Form aufweisen, so dass der Schlauch in die Führung eingedrückt werden kann.

Nach einer Weiterbildung kann dem Klemmelement ein Fortsatz zugeordnet sein.. Dieser Fortsatz kann sich vorzugsweise oberhalb der ersten Führung und/oder der zweiten Führung erstrecken, so dass ein Schlauch zwischen erster Führung und Fortsatz einklemmbar ist und/oder der zweiten Führung und Fortsatz einklemmbar ist. Dabei kann der Fortsatz einstückig mit dem Klemmelement ausgebildet sein. Ebenso kann der Fortsatz einer Pumpvorrichtung oder einem Pumpengehäuse einer Pumpvorrichtung zugeordnet sein und mit einem, mit der Pumpvorrichtung in Verbindung gebrachten Klemmelement, zusammenwirken. Durch den Fortsatz kann der Schlauch zwischen Klemmelement und Fortsatz durch Reibhaftung fixiert werden. Hierdurch wird ein Verschieben des Schlauches nach dem Einlegen desselben verhindert. Beispielsweise kann das Klemmelement L-förmig ausgestaltet sein.

Mit anderen Worten kann durch den Fortsatz ein Halten des Schlauches an einem weiteren am Umfang des Schlauches ausgebildeten Abschnitt des Fortsatzes erfolgen. Hierdurch wird die Aufnahme des Schlauches verbessert. Insbesondere kann der Fortsatz an einer, einer Führung gegenüberliegenden Stelle, ausgebildet sein, so dass ein Einklemmen des Schlauches ermöglicht wird. Der Fortsatz kann dabei sowohl an dem ersten als auch an dem zweiten Abschnitt des Klemmelements ausgebildet sein. Vorzugsweise ist der Fortsatz an dem zweiten Abschnitt ausgebildet. Ist der erste Abschnitt zu dem zweiten Abschnitt beweglich oder vorgespannt, so kann der Abstand zwischen Fortsatz und erstem Abschnitt verändert werden, beziehungsweise der Schlauch zwischen Fortsatz und erstem Abschnitt mittels der Federkraft eingespannt werden.

Weiter kann eine Pumpvorrichtung für eine medizinische Vorrichtung vorgesehen sein, welche ein Klemmelement nach einem der vorhergehenden Aspekte, aufweist.

Nach einer Weiterbildung weist die Pumpvorrichtung weiter ein Pumpengehäuse mit einem Pumpenbett, einen Rotor, welcher in dem Pumpenbett ausgebildet ist, und an welchem zumindest ein Okklusionselement angeordnet ist, wobei ein Schlauch zwischen Pumpenbett und Rotor derart einlegbar ist, dass er durch das Okklusionselement radial gegen das Pumpenbett gedrückt wird, so dass bei einer Rotation des Rotors Fluid in dem Schlauch transportierbar ist, einen Pumpeneingang, der im Pumpengehäuse ausgebildet ist, in welchem der Schlauch der Pumpvorrichtung zugeführt wird, einen Pumpenausgang, der im Pumpenbett ausgebildet ist, in welchem der Schlauch aus der Pumpvorrichtung geführt wird, auf.

Nach einer Weiterbildung kann das Klemmelement in das Pumpengehäuse derart einsetzbar sein, dass das Klemmelement einen Teil einer Schlauchführung bildet und durch das Klemmelement eine Fixierung des Schlauches am Pumpeneingang sowie am Pumpenausgang erreichbar ist.

Mit anderen Worten kann das Klemmelement einen Teilabschnitt der Schlauchführung am Pumpeneingang und Pumpenausgang ausbilden. Dabei kann ein Teil der Schlauchführung von dem Klemmelement und ein Teil der Schlauchführung am Pumpeneingang und Pumpenausgang am Pumpengehäuse ausgebildet sein. Alternativ kann die Schlauchführung am Pumpeneingang und Pumpenausgang nur durch das Klemmelement definiert werden. Beispielsweise kann das Pumpengehäuse am Pumpeneingang und/oder Pumpenausgang Vorsprünge aufweisen, welche einen Teilabschnitt der Schlauchführung bilden. Durch diese Wechselwirkung zwischen Pumpengehäuse und Klemmelement kann so eine definierte Schlauchführung erreicht werden.

Nach einer Weiterbildung kann die Schlauchführung am Pumpeneingang und/oder Pumpenausgang, vorzugsweise durch das Klemmelement, derart erfolgen, dass die erste Führung und die zweite Führung derart aufeinander zulaufen, so dass die erste Führung und die zweite Führung in Richtung des Rotors weiter beabstandet sind. Mit anderen Worten können die Führungen auf dem Klemmelement an einem Abschnitt, welcher näher an der Pumpvorrichtung, beispielsweise dem Rotor liegt, weiter beabstandet sein als an einem Abschnitt, welcher weiter weg von der Pumpvorrichtung, beispielsweise dem Rotor liegt. Ebenso oder alternativ kann die Schlauchführung am Pumpeneingang und/oder Pumpenausgang, vorzugsweise durch das Klemmelement, derart erfolgen, dass der Schlauch tangential zum Rotor zuführbar ist.

Nach einer Weiterbildung der Pumpvorrichtung für eine medizinische Vorrichtung kann der Pumpeneingang und Pumpenausgang durch das Pumpengehäuse und Klemmelement derart ausgebildet sein, dass der Schlauch am Pumpeneingang und/oder am Pumpenausgang an sich je gegenüberliegenden Seiten fixierbar ist.

Weiter kann eine Blutbehandlungsvorrichtung mit einer Pumpvorrichtung nach vorhergehenden Aspekten sowie einer Klemmvorrichtung nach vorhergehenden Aspekten vorgesehen sein.

Die Blutbehandlungsvorrichtung kann als Dialysevorrichtung, Hämodialysevorrichtung, Hämofiltrationsvorrichtung, Hämodiafiltrationsvorrichtung, Apheresevorrichtung, oder Plasmabehandlungsvorrichtung ausgebildet sein.

Ebenso kann ein Schlauchsatz, vorzugsweise für eine extrakorporale Blutbehandlung, vorzugsweise eine Dialysebehandlung, vorgesehen sein. Dabei kann ein Teilabschnitt eines Schlauches für eine extrakorporale Blutbehandlung, vorzugsweise eine Dialysebehandlung, mit einem erfindungsgemäßen Klemmelement verbunden sein. Dabei kann der Schlauch mit der ersten Führung und/oder der zweiten Führung vor dem Einsetzen in eine Blutbehandlungsvorrichtung verbunden sein. Ist der Schlauch nicht mit beiden Führungen verbunden, so kann der Schlauch nach dem Einlegen des Klemmelements in eine Blutbehandlungsvorrichtung mit der anderen Führung verbunden werden.

Durch das erfindungsgemäße Klemmelement, Pumpvorrichtung oder Blutbehandlungsvorrichtung ist es somit möglich die auf den Schlauch und das in dem Schlauch geführte Medium wirkenden Kräfte zu reduzieren. Insbesondere treten durch das schräge, beziehungsweise tangential zum Rotor der Pumpvorrichtung, Einlaufen des Schlauches in die Pumpvorrichtung geringere Kräfte auf. Dies ist insbesondere der Fall, da ein Krafteinwirkung der Pumpvorrichtung auf den Schlauch nicht sprunghaft ansteigt.

Da der Schlauch bereits tangential, oder in Umfangsrichtung des Rotors, das heißt entlang der Drehbewegung eingeführt wird, muss der Schlauch keine abrupte Richtungsänderung erfahren. Vielmehr wird der Schlauch bereits in der Umfangsrichtung eingeführt, so dass ein allmähliches Ansteigen der auf den Schlauch wirkenden Kraft stattfindet. Mit anderen Worten ist ein Anlaufen eines Okklusionselements auf dem Schlauch mit kontinuierlich zunehmender Krafteinwirkung gegeben. Die Vermeidung einer sprunghaften Krafteinwirkung wirkt sich ebenso positiv auf das im Schlauch geführte Fluid aus. Wird beispielsweise Blut in dem Schlauch geführt und kommt es zu einer sprunghaften Krafteinwirkung so kann dies zu Blutschädigungen führen. Durch die erfindungsgemäße Lehre kann eine sprunghafte Krafteinwirkung und somit Schädigungen an dem im Schlauch geführten Fluid sowie dem Schlauch selbst vermieden werden.

Zudem wird es durch das erfindungsgemäße Klemmelement ermöglicht, eine Veränderung der Einlauf- bzw. Auslaufgeometrie zu ermöglichen, ohne dabei diese Geometrie an dem Pumpengehäuse selbst vornehmen zu müssen. So ist eine kostengünstige und konstruktiv einfache Möglichkeit gegeben, bei bereits bestehenden Pumpvorrichtungen eine gewünschte Einlauf- bzw. Auslaufgeometrie für den Schlauch zu erreichen.

### Kurzbeschreibung der Zeichnungen

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren der Zeichnung gilt:
- Fig. 1: zeigt eine schematische Darstellung einer Pumpvorrichtung;
- Fig. 2: zeigt ein vereinfachtes Flussbild eines Fluidsystems einer Blutbehandlungsvorrichtung;
- Fig. 3: zeigt eine schematische Darstellung eines Teilausschnittes einer Vorderansicht einer Blutbehandlungsvorrichtung;
- Fig. 4: zeigt eine schematische Darstellung des Klemmelements in einer Seitenansicht;
- Fig. 5: zeigt eine schematische Darstellung eines Klemmelements in einer Draufsicht.

### Detaillierte Beschreibung eines Ausführungsbeispiels

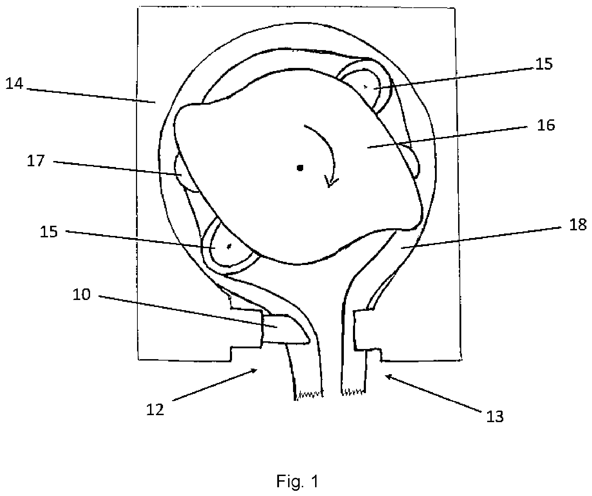

Die in Fig. 1 gezeigte Pumpvorrichtung weist ein Pumpengehäuse 14 auf. In dem Pumpengehäuse 14 sind der Rotor 16 sowie die Okklusionselemente 15 und Führungselemente 17 angeordnet. Das Pumpengehäuse 14 weist zudem ein Pumpenbett auf. In dieses Pumpenbett kann ein Schlauch 18, wie in Fig. 1 gezeigt, eingelegt werden. Der Schlauch 18 kann ein Blutschlauch, Dialysierflüssigkeitsschlauch, oder Substituatschlauch sein.

Analog hierzu kann die Pumpvorrichtung ebenso eine Schlauchpumpe oder auch peristaltische Pumpe sein und als eine Blutpumpe, Dialysierflüssigkeitsspumpe oder Substituatpumpe verwendet werden. Derartige Pumpen können in einer Blutbehandlungsvorrichtung 20 eingesetzt werden.

Fig. 1 zeigt zudem am Umfang des Rotors 16 zusätzlich axiale Führungselemente 17. Die Führungselemente 17 dienen der Ausrichtung des Schlauches und verhindern insbesondere ein Herausrutschen des Schlauches aus dem Schlauchbett. Die axialen Führungselemente sind dabei vorzugsweise drehbar angebracht, so dass zwischen Führungselementen 17 und Schlauch keine Reibung entsteht. Wie dargestellt, können zwei Führungselemente 17 am Rotor 16 angebracht sein. Ebenso können mehrere, etwa drei oder vier, oder auch nur ein Führungselement 17 am Rotor 16 vorgesehen sein.

Auf der Vorderseite des Rotors 16 ist eine Abdeckung vorgesehen. Ebenso kann der Rotor einen aus dem Rotor 16 ausklappbaren Handgriff aufweisen, hier nicht dargestellt. Mittels dieses Handgriffes kann der Rotor 16 auch per Hand rotiert, und die Pumpe die Pumparbeiten leisten. In der in Fig. 1 gezeigten Pumpvorrichtung ist das Klemmelement 1 bereits in die Pumpvorrichtung eingesetzt. Aus dieser Perspektive ist daher nur ein Fortsatz 10 des Klemmelements 1 zu sehen. Dieser Fortsatz 10 dient der Befestigung des Schlauches in einer Richtung aus der Ebene heraus. Wie in Fig. 1 zu sehen ist, wird durch das Klemmelement 1 eine zum Rotor 16 tangentiale Zuführung des Schlauches ermöglicht. Das Okklusionselement 15 bewirkt daher keine sprunghafte Krafteinwirkung auf den Schlauch. Vielmehr wird die Krafteinwirkung langsam nach dem ersten Kontakt zwischen Okklusionselement 15 und Schlauch erhöht. Hierdurch können Blutschädigungen, welche durch einen sprunghaften Druckanstieg im Schlauch verursacht werden können, vermieden. Ebenso werden durch die tangentiale Einführung des Schlauches Scherkräfte, verursacht durch eine gezwungene starke Richtungsänderung durch das auf den Schlauch treffende Okklusionselement, vermieden.

Zudem zeigt Fig. 1 am Rotor 16 angebrachte Okklusionselemente 15. Diese Okklusionselemente 15 sind hier zylindrisch in Form von Rollen ausgebildet. Am Umfang des Rotor sind beispielhaft zwei Okklusionselemente 15 ausgebildet. Ebenso können aber auch drei oder vier Okklusionselemente oder nur ein Okklusionselement vorgesehen sein. Die Okklusionselemente 15 sind derart ausgebildet, dass sie den Schlauch gegen das Pumpenbett radial nach außen drücken. Die Okklusionselemente können hierzu mittels einer Feder vorgespannt sein. Durch das Anpressen der Okklusionselemente15 gegen den Schlauch sowie die Rotation des Rotors 16 wird somit Fluid in dem Schlauch gefördert.

Ist ein Schlauch in die Pumpvorrichtung eingelegt und dreht die Pumpvorrichtung, wie in Fig. 1 gezeigt, im Uhrzeigersinn, so wird das Fluid im Schlauch von dem Pumpeneingang 12 zu dem Pumpenausgang 13 gefördert. Im Falle einer Blutpumpe kann so Blut von dem Patienten gezogen und über die arterielle Leitung mittels der Blutpumpe einem Dialysator 21 zugeführt werden. Der Dialysator 21 weist eine Blutkammer auf, welche mit der venösen und arteriellen Blutleitung verbunden ist, sowie eine Dialysierflüssigkeitskammer. Diese Kammern sind durch eine semi-permeable Membran voneinander getrennt. Im Dialysator 21 selbst fließt das Blut im Gegenstromprinzip, wobei Dialysierflüssigkeit auf der anderen Seite strömt. Die Blutseite des Dialysators 21 sowie die Blutpumpe sind Teil des extrakorporalen Blutkreislaufs.

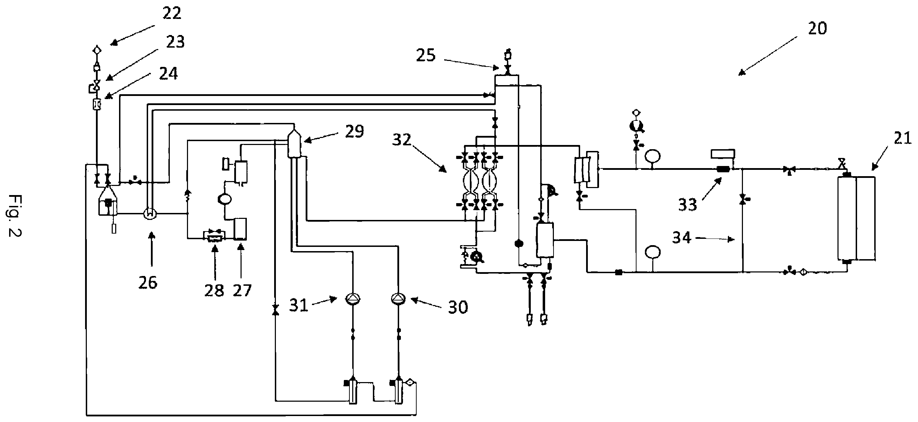

Fig. 2 zeigt ein vereinfachtes Flussbild eines Fluidsystems einer Blutbehandlungsvorrichtung 20 als Dialysemaschine. Die Flüssigkeitsversorgung der Blutbehandlungsvorrichtung 20 erfolgt über einen Dialysewasseranschluss 22, ein nachgeschaltetes Druckminderventil 23, welches den Druck beispielsweise auf etwa 0,5 bar reduziert, sowie eine Zulaufdrossel 24. Über den Dialysewasseranschluss 1 wird Permeat, das heißt enthärtetes und filtriertes Wasser, zugeführt.

Nach dem Passieren des Dialysators 21 wird das Dialysat über eine Dialysierflüssigkeitsabführleitung und ein Abflussventil 25 einem Abfluss zugeführt. Dabei kann eine Erwärmung der frischen Dialysierflüssigkeit über einen Wärmetauscher 26 durch das Dialysat stattfinden, um anschließend etwa mittels einer Heizspirale oder eines Heizstabs weiter erwärmt zu werden. Zudem wird das Permeat einer Entgasung in einer Entgasungskammer 27 unterzogen. Um eine Entfernung von Luft aus dem Permeat zu fördern, wird das Permeat hierfür mittels einer Entgasungsdrossel 28 einem Unterdruck ausgesetzt. Durch die Temperaturerhöhung und Druckverringerung kann damit Luft in Blasenform über einen nachgeschalteten Luftabscheider 29 entweichen.

Nach der Entgasung des Permeats wird durch die Zumischung mindestens einer Konzentratlösung das Mischfluid, hier die Dialysierflüssigkeit, erzeugt. Zur Bereitstellung der frischen Dialysierflüssigkeit wird somit Permeat, zugeführt über den Dialysewasseranschluss 22, sowie beispielsweise zwei Konzentratlösungen, etwa einer Bicarbonatkonzentratlösung und einer Säurekonzentratlösung, zugeführt beispielsweise aus hier nicht dargestellten Konzentratbehältern, und anschließend vermischt. Dabei können die Konzentratlösungen über Konzentratpumpen 30, 31 gefördert werden. Die Konzentratpumpen 30, 31 können beispielsweise als Hubkolbenpumpen, Membranpumpen oder Zahnradpumpen ausgebildet sein. Die Proportionierung, das heißt die Mischung von Säurekonzentrat und Bicarbonat mit Permeat in einem vorbestimmten Verhältnis, kann volumetrisch oder leitfähigkeitsgesteuert erfolgen. Bei der in diesem Ausführungsbeispiel gezeigten volumetrischen Proportionierung wird das zugeführte Volumen über eine getaktete Zuführung mittels der Konzentratpumpen 30, 31, beispielsweise Hubkolbenpumpen, erreicht.

Die durch die Vermischung entstandene Dialysierflüssigkeit durchströmt anschließend einen Teil einer Bilanzkammer 32 und gelangt damit in den Dialysierflüssigkeitskreislauf. Die Bilanzkammer 32 bilanziert dabei zwischen der frischen Dialysierflüssigkeit und der verbrauchten Dialysierflüssigkeit, dem Dialysat. Dem Dialysator 21 vorgeschaltet ist ein Mischfluidsensor 33, um die richtige Zusammensetzung der Dialysierflüssigkeit zu überprüfen. Dem Mischfluidsensor 33, beispielsweise als Leitfähigkeitssensor ausgebildet, ist ein Bypass-Ventil 34 nachgeschaltet. Detektiert der Mischfluidsensor 33 während der Dialysebehandlung eine unphysiologische Flüssigkeit, das heißt eine Flüssigkeit, welche einer vorgegebenen Bedingung, beispielsweise einer vorgegebenen Leitfähigkeit, nicht erfüllt, wird das Bypass-Ventil 34 geöffnet und die Dialysierflüssigkeit über einen Leitungsabschnitt 34 geleitet.

Die Aktoren, Pumpen und Ventile der Blutbehandlungsvorrichtung können mit einer hier nicht dargestellten Steuervorrichtung verbunden sein, bzw. in Kommunikation stehen.

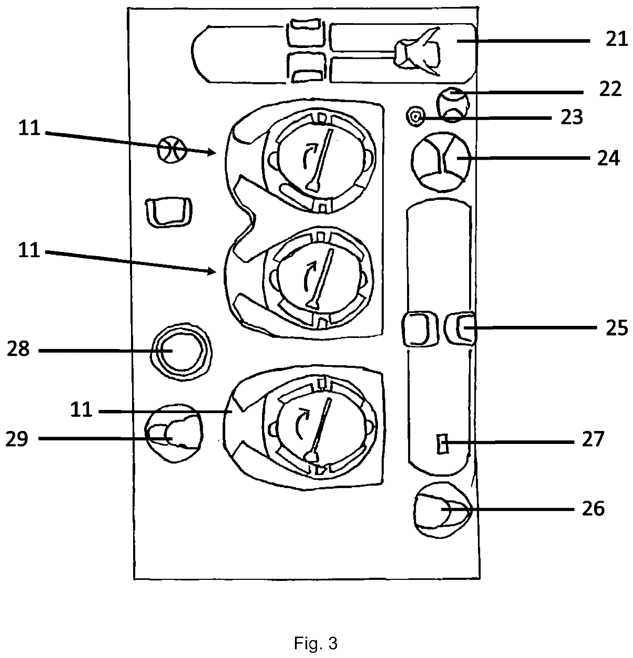

Fig. 3 zeigt einen Teilausschnitt einer Vorderansicht einer extrakorporalen Blutbehandlungsvorrichtung 20. Dabei kann der Blutkreislauf ein Schlauchsystem aufweisen, durch welches während der Behandlung das Blut des Patienten strömen kann. Das Schlauchsystem kann zur Behandlung in eine Dialysemaschine eingelegt werden und mit Modulen, beispielsweise Blutmodulen der Dialysevorrichtung, wechselwirken. Dabei kann das Blutmodul eine arterielle Patientenschlauchklemme 29 eines arteriellen Abschnitts einer arteriellen Blutleitung aufweisen. Wie beschrieben wird das Blut einem Patienten mittels einer arteriellen Konnektionsnadel entzogen und mittels einer venösen Konnektionsnadel zurückgeführt. Entsprechend kann das Blutmodul ebenso eine venöse Patientenschlauchklemme 26 aufweisen, wobei das Blut stromab dieser Patientenschlauchklemme 26 dem Patienten über die venöse Konnektionsnadel wieder zugeführt wird.

Der in Fig. 3 gezeigte Teilausschnitt der Blutbehandlungsvorrichtung 20 weist von oben nach unten drei Schlaupumpen 11 auf, wobei die erste Pumpe eine Single-Needle-Pumpe ist, die zweite Pumpe eine Blutpumpe, sowie die dritte Pumpe eine Substituatpumpe. Eine extrakorporale Blutbehandlungsvorrichtung kann dabei alle drei Schlauchpumpen aufweisen, aber auch nur eine Blutpumpe oder nur eine Blutpumpe und eine Substituatpumpe. Mittels der Substituatpumpe kann Substituat per Prädilution oder Postdilution der arteriellen, bzw. venösen Blutleitung zugeführt werden.

Zudem können eine Zugabestelle für Heparin oder ein anderes Antikoagulans vorgesehen sein. So kann die Blutbehandlungsvorrichtung eine Heparinpumpe 21, eine arterielle Druckmesseinheit 28 und Sensoren 25, 27 aufweisen. Diese Sensoren 25, 27 überwachen den Schlauch auf Luftblasen sowie auf den Inhalt. So kann ein Ultraschallsensor sowie ein optischer Sensor zur Farberkennung vorgesehen sein. Schließlich weist die Blutbehandlungsvorrichtung eine arterielle Druckmesseinheit 28 sowie einen venösen Druckanschluss 23 auf.

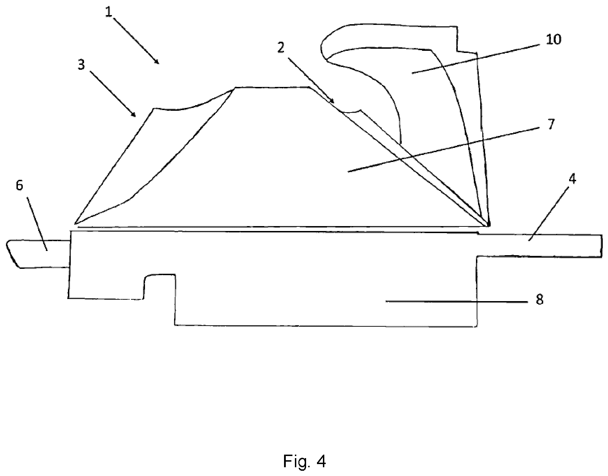

Fig. 4 zeigt ein erfindungsgemäßes Klemmelement 1 in einer Seitenansicht. Das Klemmelement kann mit einer der zuvor beschriebenen Schlauchpumpen 11 verbunden werden. Insbesondere kann durch das Einsetzen des Klemmelements 1 in das Pumpengehäuse 14 die Schlauchführung am Pumpeneingang 12 sowie Pumpenausgang 13 gebildet werden. Hierzu wird das Klemmelement 1 über ein Verbindungselement 6 (wie in Fig. 4 dargestellt einen Vorsprung) mit dem Pumpengehäuse 14 verbunden. Hierzu kann in dem Pumpengehäuse 14 eine entsprechende Vertiefung oder Nut ausgebildet sein. Alternativ kann an dem Pumpengehäuse 14 ein Vorsprung und an dem Klemmelement 1 als Verbindungselement 6 eine entsprechende Vertiefung oder Nut ausgebildet sein. Mit anderen Worten kann eine formschlüssige Verbindung zwischen Klemmelement 1 und Pumpengehäuse 14 ausgebildet werden. Ebenso kann eine magnetische Verbindung zwischen Klemmelement 1 und Pumpengehäuse 14 ausgebildet sein.

Zur einfachen Montage wird das Klemmelement 1 zuerst mit dem zweiten Seitenabschnitt 5, an welchem das Verbindungselement 6 ausgebildet ist, schräg in das Pumpengehäuse 14 eingeführt. Anschließend kann das Klemmelement 1 durch ein einfaches Eindrücken vollständig eingesetzt werden. Dies ist insbesondere möglich, da der zweite Seitenabschnitt 4 ein elastisches Element aufweisen kann. Hierzu kann, wie in Fig. 4 dargestellt, an dem zweiten Seitenabschnitt 4 eine elastische Seitenplatte ausgebildet sein. Diese kann insbesondere aus einem elastischen Kunststoff ausgebildet sein. Mit anderen Worten kann ein thermoplastisches Elastomer sowie Silikon oder Gummi eingesetzt werden.

Zur Demontage des Klemmelements 1 aus dem Pumpengehäuse 14 kann entsprechend das Klemmelement über einen Fortsatz 10 gegriffen, und in Richtung des zweiten Seitenabschnitts 5 gedrückt werden. Anschließend kann das Klemmelement 1 mit dem ersten Seitenabschnitt 5 mit dem elastischen Element zuerst angehoben und anschließend vollständig aus dem Pumpengehäuse 14 entnommen werden.

Der an dem Klemmelement 1 ausgebildete Fortsatz 10 ist insbesondere oberhalb der ersten Führung 2 ausgebildet. Zusätzlich oder alternativ kann ein Fortsatz auch oberhalb der zweiten Führung 3 ausgebildet sein. Der Fortsatz 10 kann sich von einem ersten Abschnitt 7 oder zweiten Abschnitt 8 des Klemmelements 1 erstrecken. Vorzugsweise erstreckt sich der Fortsatz 10 von dem zweiten Abschnitt 8, welcher im eingebauten Zustand des Klemmelements 1, relativ zu dem Pumpengehäuse **14** unbeweglich ausgebildet ist. Der Fortsatz 10 ist insbesondere dazu geeignet einen Schlauch zu zumindest zwei Seiten zu fixieren.

So kann der Fortsatz den Schlauch von einer Oberseite und die erste Führung 2, bzw. zweite Führung 3, den Schlauch von einer Unterseite fixieren. Der Schlauch kann von einer Seite, in Bezug zu dem in Fig. 4 gezeigten Ausführungsbeispiel, von der linken Seite eingelegt werden, und von einer Oberseite, das heißt einer von dem Pumpengehäuse 14 wegführenden Richtung gehalten werden. Ebenso oder alternativ kann der Schlauch von drei Punkten oder flächenförmig fixiert werden. Dabei kann der Fortsatz 10 auf einer Unterseite, welche zu der ersten Führung 2, bzw. zweiten Führung 3, zeigt, eine Bogenform aufweisen. Der Radius der Bogenform kann dabei dem Radius eines üblichen Schlauches entsprechen. Insbesondere kann der Radius 5mm bis 30mm, insbesondere 10mm bis 20mm, aufweisen.

Die erste Führung 2 und/oder die zweite Führung 3 können halbkreisförmig oder größer 180° im Querschnitt ausgebildet sein. Ebenso können die erste Führung 2 und/oder die zweite Führung 3 etwa nur Viertel-kreisförmig oder mehr ausgebildet sein. Insbesondere können die erste Führung 2 und/oder die zweit Führung 3 eine Bogenform von 20° bis 270°, vorzugsweise von 30° bis 180°, noch bevorzugter von 35° bis 90°, noch bevorzugter etwa 45°, ausgebildet sein.

Das in Fig. 4 gezeigte Klemmelement 1 kann dabei nach dem Einlegen in das Pumpengehäuse 14 mit einem Schlauch verbunden werden. Insbesondere kann nach dem Einlegen der Schlauch in die erste Führung 2 und die zweite Führung 3 eingelegt werden. Alternativ kann ein in Fig. 4 nicht dargestellter Schlauch bereits vor dem Einlegen mit dem Klemmelement 1 verbunden sein. Dabei kann das Klemmelement Teil eines Schlauchsets, oder Schlauchsatzes, sein. Hierbei kann der Schlauch samt Klemmelement als Disposable ausgebildet sein.

Beispielsweise kann der Schlauch in einer Art Schlaufe mit der ersten Führung 2 und der zweiten Führung 3 bereits vor dem Einlegen in eine Pumpgehäuse 14 verbunden sein. Alternativ kann der Schlauch auch nur mit einer der Führungen 2, 3 vor dem Einlegen verbunden sein und nach dem Einlegen in das Pumpengehäuse 14 mit der anderen Führung 2, 3 verbunden werden. Ist der Schlauch bereits vor dem Einlegen mit dem Klemmelement 1 verbunden, so wird hierdurch die Handhabung erleichtert. Insbesondere sind durch das Klemmelement 1 mit bereits vor dem Einlegen eingesetzten Schlauch, die Handhabungsschritte reduziert. Dies ist insbesondere vorteilhaft, da die Zeit zum Aufrüsten einer Dialysevorrichtung nur eine bestimmte Zeit in Anspruch nehmen darf, ohne die strenge Zeitplanung zu gefährden.

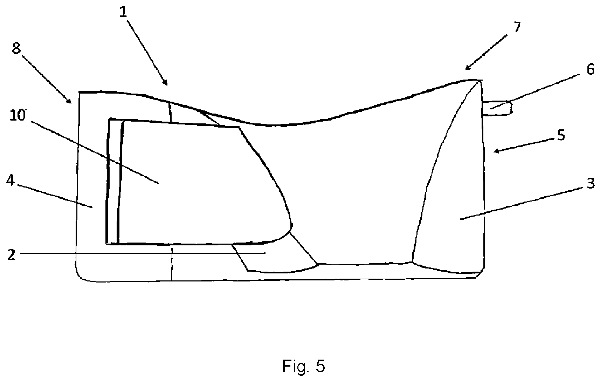

Fig. 5 zeigt das Klemmelement 1 schematisch in einer Draufsicht. Dabei ist die Form der Führungen 2, 3 auf eine Ebene projiziert, nahezu dreieckförmig. Der Vorsprung 6, zur Verbindung des Klemmelements 1 mit der Pumpvorrichtung, ist in der hier gezeigten Ausführungsform am Rand des Klemmelements 1 ausgebildet. Alternativ kann der Vorsprung 6 aber auch mittig oder mehrere Vorsprünge 6 an verschiedenen Stellen ausgebildet sein.

Das Klemmelement 1 kann weiter eine hier nicht dargestellte Drehachse aufweisen. Die Drehachse kann dabei im zweiten Abschnitt 8 ausgebildet sein. Ebenso kann der zweite Abschnitt 8 eine hier nicht dargestellte Feder aufweisen. Diese Feder kann als Spiralfeder auf der Drehachse gelagert sein. Der erste Abschnitt 7 kann um diese Drehachse drehbar mit dem zweiten Abschnitt 8 verbunden sein. So kann ein Vorspannen eines in die Führungen 2, 3 eingelegten Schlauches gegen den Fortsatz 10 oder Elemente des Pumpengehäuses erreicht werden.

## Patentansprüche

1. Klemmelement (1) zum Fixieren eines Schlauches, aufweisend eine erste Führung (2) und eine zweite Führung (3) zum Aufnehmen eines Schlauches,
wobei die zwei Führungen (2, 3) aufeinander zulaufen, und das Klemmelement (1) derart ausgebildet ist, dass es mit einer Pumpvorrichtung (11) verbindbar ist, so dass eine Schlauchführung durch die zwei Führungen an einem Pumpeneingang (12) und einem Pumpenausgang (13) der Pumpvorrichtung (11) ausbildbar ist,
**dadurch gekennzeichnet dass**
das Klemmelement zumindest einen ersten Abschnitt und einen zweiten Abschnitt aufweist, welche zueinander beweglich ausgebildet sind,
wobei die zwei Führungen (2, 3) auf dem ersten Abschnitt (7) ausgebildet sind, wobei der erste Abschnitt (7) vorzugsweise um eine Drehachse des zweiten Abschnitts (8) rotierbar ist,
wobei der erste Abschnitt (7) vorzugsweise mittels einer Feder, insbesondere einer Spiralfeder, in einer ersten Position vorgespannt ist,
wobei in der ersten Position der erste Abschnitt (7) von dem zweiten Abschnitt (8) zumindest teilweise beabstandet ist.

2. Klemmelement nach Anspruch 1, wobei das Klemmelement (1) zumindest teilweise elastisch ausgebildet ist, insbesondere ein erster Seitenabschnitt (4) des Klemmelements (1) elastisch ausgebildet ist.

3. Klemmelement nach Anspruch 1 oder 2, wobei das Klemmelement (1) zumindest ein Verbindungselement (6) zum Verbinden mit einer Pumpvorrichtung, insbesondere einen Vorsprung, insbesondere einen stiftförmigen Vorsprung, aufweist, wobei dieses Verbindungselement (6) vorzugsweise an einem zweiten Seitenabschnitt (5), welcher dem ersten Seitenabschnitt (4) gegenüberliegt, ausgebildet ist.

4. Klemmelement zum Fixieren eines Schlauches nach zumindest einem der Ansprüch 1 bis 3, wobei eine Rotationsachse, um die der erste Abschnitt (7) rotierbar ist, an dem zweiten Abschnitt (8) angeordnet ist.

5. Klemmelement zum Fixieren eines Schlauches nach Anspruch 4, wobei die Rotationsachse an dem zweiten Abschnitt (8) und die Spiralfeder auf der Rotationsachse gelagert ist.

6. Klemmelement zum Fixieren eines Schlauches nach zumindest einem der Ansprüche 1 bis 5, wobei die erste und/oder zweite Führung (2, 3) rillenförmig ist und/oder wobei die erste Führung (2) und/oder die zweite Führung (3) den Schlauch an zwei gegenüberliegenden Seiten des Schlauchs aufnimmt.

7. Klemmelement zum Fixieren eines Schlauches nach zumindest einem der Ansprüche 1 bis 6, wobei dem Klemmelement zumindest ein Fortsatz (10) zugeordnet ist, welcher sich oberhalb der ersten Führung (2) und/oder der zweiten Führung (3) erstreckt, so dass ein Schlauch zwischen erster Führung (2) und Fortsatz (10) und/oder zweiter Führung (3) und Fortsatz (10) einklemmbar ist.

8. Pumpvorrichtung für eine medizinische Vorrichtung, aufweisend ein Klemmelement (1) nach zumindest einem der Ansprüche 1 bis 7.

9. Pumpvorrichtung nach Anspruch 8, weiter aufweisend
ein Pumpengehäuse (14) mit einem Pumpenbett (12),
einen Rotor (16), welcher in dem Pumpenbett (12) angeordnet ist, und an welchem zumindest ein Okklusionselement (15) angeordnet ist,
wobei ein Schlauch zwischen Pumpenbett (12) und Rotor (16) derart einlegbar ist, dass er durch das Okklusionselement (15) radial gegen das Pumpenbett (12) gedrückt wird, so dass bei einer Rotation des Rotors (16) Fluid in dem Schlauch transportierbar ist,
einem Pumpeneingang (12), der im Pumpengehäuse (14) ausgebildet ist, in welchem der Schlauch der Pumpvorrichtung zugeführt wird, und
einem Pumpenausgang (13), der im Pumpenbett ausgebildet ist, in welchem der Schlauch aus der Pumpvorrichtung geführt wird.

10. Pumpvorrichtung nach Anspruch 8 oder 9 wobei das Klemmelement (1) in das Pumpengehäuse (14) derart einsetzbar ist, dass das Klemmelement (1) einen Teil einer Schlauchführung bildet und durch das Klemmelement (1) eine Fixierung des Schlauches am Pumpeneingang (12) sowie am Pumpenausgang (13) erreichbar ist.

11. Pumpvorrichtung nach zumindest einem der Ansprüche 8 bis 10, wobei die Schlauchführung am Pumpeneingang (12) und/oder Pumpenausgang (13), vorzugsweise durch das Klemmelement, derart erfolgt, dass die erste Führung (2) und die zweite Führung (3) derart aufeinander zulaufen, so dass die erste Führung (2) und die zweite Führung (3) in Richtung des Rotors (16) weiter beabstandet sind, so dass ein Schlauch vorzugsweise tangential zum Rotor (16) zuführbar ist.

12. Pumpvorrichtung nach zumindest einem der Ansprüche 8 bis 11, wobei der Pumpeneingang (12) und der Pumpenausgang (13) durch das Pumpengehäuse (14) und Klemmelement (1) derart ausgebildet sind, dass ein Schlauch am Pumpeneingang (12) und/oder am Pumpenausgang (13) an zumindest zwei Schlauchseiten, insbesondere an zumindest zwei sich gegenüberliegenden Schlauchseiten, fixierbar ist.

13. Blutbehandlungsvorrichtung mit einem einer Pumpvorrichtung nach zumindest einem der Ansprüche 8 bis 12.

14. Schlauchsatz mit einem Klemmelement nach einem der Ansprüche 1 bis 7 vorzugsweise zur Verwendung bei einer Pumpvorrichtung nach einem der Ansprüche 8 bis 12.

## Claims

1. A clamping element (1) for securing a tube, having a first guide (2) and a second guide (3) for receiving a tube,
wherein the two guides (2, 3) run towards each other, and the clamping element (1) is designed in such a manner that it can be connected to a pump device (11) such that a tube guide can be formed by the two guides at a pump inlet (12) and a pump outlet (13) of the pump device (11),
**characterized in that**
the clamping element has at least a first portion and a second portion which can be moved relative to each other, wherein the two guides (2, 3) are formed on the first portion (7), wherein the first portion (7) can preferably be rotated about an axis of rotation of the second portion (8),
wherein the first portion (7) is preferably biased in a first position by means of a spring, in particular a spiral spring, wherein in the first position the first portion (7) is at least partially spaced apart from the second portion (8).

2. The clamping element according to claim 1, wherein the clamping element (1) is at least partially elastic, in particular a first side portion (4) of the clamping element (1) is elastic.

3. The clamping element according to claim 1 or 2, wherein the clamping element (1) has at least one connecting element (6) for connecting to a pump device, in particular a protrusion, in particular a pin-shaped protrusion, wherein this connecting element (6) is preferably formed on a second side portion (5) which lies opposite the first side portion (4).

4. The clamping element for securing a tube according to at least one of claims 1 to 3, wherein an axis of rotation about which the first portion (7) can be rotated is arranged on the second portion (8).

5. The clamping element for securing a tube according to claim 4, wherein the axis of rotation is mounted on the second portion (8) and the spiral spring is mounted on the axis of rotation.

6. The clamping element for securing a tube according to at least one of claims 1 to 5, wherein the first and/or second guide (2, 3) is groove-shaped and/or wherein the first guide (2) and/or the second guide (3) receives the tube on two opposite sides of the tube.

7. The clamping element for securing a tube according to at least one of claims 1 to 6, wherein the clamping element is assigned at least one extension (10) which extends above the first guide (2) and/or the second guide (3) such that a tube can be clamped between the first guide (2) and the extension (10) and/or the second guide (3) and the extension (10).

8. A pump device for a medical device, having a clamping element (1) according to at least one of claims 1 to 7.

9. The pump device according to claim 8, further having a pump housing (14) with a pump bed (12),
a rotor (16) which is arranged in the pump bed (12) and on which at least one occlusion element (15) is arranged,
wherein a tube can be inserted between the pump bed (12) and the rotor (16) in such a way that it is pressed radially against the pump bed (12) by the occlusion element (15), such that fluid can be transported in the tube when the rotor (16) rotates,
a pump inlet (12) which is formed in the pump housing (14) into which the tube of the pump device is fed, and
a pump outlet (13) which is formed in the pump bed, in which the tube is fed out of the pump device.

10. The pump device according to claim 8 or 9, wherein the clamping element (1) can be inserted into the pump housing (14) in such a way that the clamping element (1) forms part of a tube guide and the clamping element (1) enables the tube to be fixed at the pump inlet (12) and at the pump outlet (13).

11. The pump device according to at least one of claims 8 to 10, wherein the tube guide at the pump inlet (12) and/or pump outlet (13) takes place, preferably by the clamping element, in such a way that the first guide (2) and the second guide (3) run towards each other in such a way that the first guide (2) and the second guide (3) are spaced apart further in the direction of the rotor (16), such that a tube can preferably be supplied tangentially with respect to the rotor (16).

12. The pump device according to at least one of claims 8 to 11, wherein the pump inlet (12) and the pump outlet (13) are formed by the pump housing (14) and clamping element (1) in such a way that a tube can be secured at the pump inlet (12) and/or at the pump outlet (13) on at least two tube sides, in particular on at least two opposite tube sides.

13. A blood treatment device having a pump device according to at least one of claims 8 to 12.

14. A tube set having a clamping element according to any one of claims 1 to 7, preferably for use in a pump device according to one of claims 8 to 12.

## Revendications

1. Élément de serrage (1) pour fixer un tuyau, comprenant un premier guide (2) et un second guide (3) pour recevoir un tuyau,
dans lequel les deux guides (2, 3) convergent l'un vers l'autre, et l'élément de serrage (1) est conçu de telle sorte qu'il peut être relié à un dispositif de pompe (11) de façon qu'un guidage de tuyau peut être formé à travers les deux guides à une entrée de pompe (12) et à une sortie de pompe (13) du dispositif de pompe (11),
**caractérisé en ce que**
l'élément de serrage comporte au moins une première section et une seconde section, lesquelles sont mobiles l'une par rapport à l'autre, dans lequel les deux guides (2, 3) sont formés sur la première section (7), dans lequel la première section (7), peut de préférence tourner autour d'un axe de rotation de la seconde section (8),
dans lequel la première section (7), est de préférence précontrainte dans une première position au moyen d'un ressort, en particulier d'un ressort hélicoïdal, dans lequel, dans la première position, la première section (7) est au moins partiellement espacée de la seconde section (8).

2. Élément de serrage selon la revendication 1, l'élément de serrage (1) étant au moins partiellement élastique, en particulier une première section latérale (4) de l'élément de serrage (1) étant élastique.

3. Élément de serrage selon la revendication 1 ou 2, l'élément de serrage (1) comportant au moins un élément de liaison (6) destiné à être relié au dispositif de pompe, en particulier une protubérance, en particulier une protubérance en forme de broche, dans lequel ledit élément de liaison (6), est de préférence formé sur une seconde section latérale (5) opposée à la première section latérale (4).

4. Élément de serrage pour fixer un tuyau selon l'au moins une quelconque des revendications 1 à 3, dans lequel un axe de rotation, autour duquel la première section (7) peut tourner, est disposé sur la seconde section (8).

5. Élément de serrage pour fixer un tuyau selon la revendication 4, dans lequel l'axe de rotation est monté sur la seconde section (8) et le ressort hélicoïdal est monté sur l'axe de rotation.

6. Élément de serrage pour fixer un tuyau selon l'au moins une quelconque des revendications 1 à 5, dans lequel le premier et/ou le second guide (2, 3) sont rainurés et/ou dans lequel le premier guide (2) et/ou le second guide (3) reçoivent le tuyau sur deux côtés opposés du tuyau.

7. Élément de serrage pour fixer un tuyau selon l'au moins une quelconque des revendications 1 à 6, l'élément de serrage étant associé à au moins une extension (10), laquelle s'étend au-dessus du premier guide (2) et/ou du second guide (3), de telle sorte que le tuyau peut être serré entre le premier guide (2) et l'extension (10) et/ou entre le second guide (3) et l'extension (10).

8. Dispositif de pompe pour un dispositif médical, présentant un élément de serrage (1) selon l'au moins une quelconque des revendications 1 à 7.

9. Dispositif de pompe selon la revendication 8, comprenant en outre un corps de pompe (14) comportant un lit de pompe (12),
un rotor (16), lequel est disposé dans le lit de pompe (12) et sur lequel est disposé au moins un élément d'occlusion (15),
dans lequel un tuyau peut être inséré entre le lit de pompe (12) et le rotor (16), de telle sorte qu'il peut être pressé radialement contre le lit de pompe (12) au moyen de l'élément d'occlusion (15), de façon qu'un fluide peut être transporté dans le tuyau lorsque le rotor (16) tourne,
une entrée de pompe (12), qui est formée dans le corps de pompe (14), dans laquelle est introduit le tuyau du dispositif de pompe, et
une sortie de pompe (13), qui est formée dans le lit de pompe, dans laquelle le tuyau est guidé hors du dispositif de pompe.

10. Dispositif de pompe selon la revendication 8 ou 9, dans lequel l'élément de serrage (1) peut être inséré dans le corps de pompe (14), de telle sorte que l'élément de serrage (1) forme une partie d'un guidage de tuyau et de telle sorte que l'élément de serrage (1) permet de fixer le tuyau à l'entrée de pompe (12) et à la sortie de pompe (13).

11. Dispositif de pompe selon l'au moins une quelconque des revendications 8 à 10, dans lequel le guidage de tuyau à l'entrée de pompe (12) et/ou à la sortie de pompe (13), de préférence au moyen de l'élément de serrage, est effectué de telle sorte que le premier guide (2) et le second guide (3) convergent l'un vers l'autre, de telle sorte que le premier guide (2) et le second guide (3) sont de préférence plus espacés dans la direction du rotor (16), de façon que le tuyau peut de préférence être acheminé tangentiellement au rotor (16).

12. Dispositif de pompe selon l'au moins une quelconque des revendications 8 à 11, dans lequel l'entrée de pompe (12) et la sortie de pompe (13) sont formées au moyen du corps de pompe (14) et de l'élément de serrage (1), de telle sorte que le tuyau peut être fixé à l'entrée de pompe (12) et/ou à la sortie de pompe (13) sur au moins deux côtés du tuyau, en particulier sur au moins deux côtés opposés du tuyau.

13. Dispositif de traitement du sang comportant un dispositif de pompe selon l'au moins une quelconque des revendications 8 à 12.

14. Ensemble tuyau comportant un élément de serrage selon l'une quelconque des revendications 1 à 7, de préférence destiné à être utilisé dans un dispositif de pompe selon l'une quelconque des revendications 8 à 12.
